(19) Europäisches Patentamt
European Patent Office
Office européen des brevets

(11) Veröffentlichungsnummer: **0 444 514 A2**

# (12) EUROPÄISCHE PATENTANMELDUNG

(21) Anmeldenummer: **91102444.6**

(22) Anmeldetag: **20.02.91**

(51) Int. Cl.5: **C08J 7/12**, G01N 33/547, G01N 33/548, C12N 11/06, C12N 11/08, C12N 11/10

(30) Priorität: **25.02.90 DE 4005927**

(43) Veröffentlichungstag der Anmeldung: **04.09.91 Patentblatt 91/36**

(84) Benannte Vertragsstaaten: **AT BE CH DE DK ES FR GB IT LI NL SE**

(71) Anmelder: **RÖHM GMBH**
**Kirschenallee**
**W-6100 Darmstadt(DE)**

(72) Erfinder: **Schmitt, Stefan, Dr.**
**Schweizertalstrasse 16**
**W 6900 Heidelberg(DE)**

## (54) Immobilisierung von Proteinen an Trägern.

(57) Diese Erfindung betrifft modifizierte, feste Träger zur kovalenten Immobilisierung von Proteinen auf Basis von an sich bekannten Matrixmaterialien, wobei die modifizierten festen Träger T eine Mehrzahl von zur kovalenten Immobilisierung geeigneten funktionellen Gruppen der Formel I

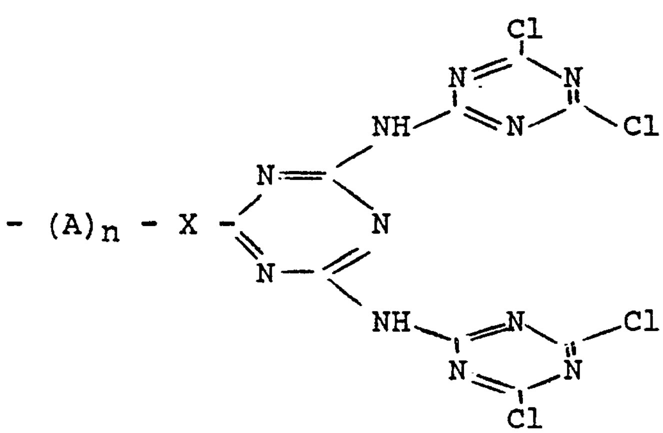

(I)

worin A für eine Spacergruppe, X für O, S, oder NH und n für null oder eins steht, besitzen, welche kovalent an das Matrixmaterial gebunden sind.

EP 0 444 514 A2

Gegenstand der Erfindung

Die Erfindung betrifft modifizierte feste Träger zur Immobilisierung von Proteinen und ein Verfahren zur Bindung von Proteinen.

Stand der Technik

Die Immobilisierung von biologisch signifikanten Proteinen an Trägern stellen ein hervorragendes Instrument sowohl in der biochemischen Forschung als in der Biotechnologie dar (c.f. Ullmann's Encyclopedia of Industrial Chemistry 5th Ed. Vol. A14, pp. 2 - 42, VCH 1989)
Als feste organische Matrixmaterialien haben bisher vor allem (modifizierte) Polysaccharide, Proteine und Aktivkohle aus natürlichem Vorkommen, so dann synthetische Polymere wie Polystyrol, Poly(meth)acrylate, Polyacrylamide, Maleinanhydrid-Polymere, Vinyl- und Allyl-Polymere und Polyamide Anwendung gefunden. Als anorganische Träger werden Mineralien, insbesondere Tonmaterialien, Kieselgur und Talk sowie synthetische Materialien wie Glas, Metalloxide und Metalle angewendet. Von den möglichen Arten der Fixierung greift die kovalente am ehesten in die aktivitätsbestimmenden Strukturen der zu fixierenden biologisch aktiven Substanzen, die überwiegend Proteine darstellen, ein. Die Trägermaterialien besitzen gewöhnlich selbst keine reaktiven Gruppen, um sie an die aktiven Gruppen der Proteine (speziell Amino-, Hydroxyl- bzw. Phenol-, Amide-, Thiol-, Carboxyl-Gruppen) anzukuppeln, sondern sie bedürfen der Aktivierung. Zur Aktivierung der Trägermaterialien ist eine ganze Reihe von reaktiven Gruppen eingeführt worden, darunter solche, die eine Alkylierung bzw. Arylierung freier Amino-, Phenol- und/oder Thiolgruppen der betreffenden biologisch aktiven Proteine bewirken (vgl. Ullmann's Encyclopedia loc.cit; US-A 4 829 101; CA-A 1 212 058). In diese Reihe gehört z.B. die polymergebundene Chlor-s-Triazinylgruppe, die - vor allem an Cellulose gebunden - zur unmittelbaren Kopplung von Proteinen dient (vgl. B.P. Surinev et.a. Biokhimiya 31, 387 (1966); G.Kay et al. Nature 216 (1967), 514; Biochim. Biophys. Acta 198 (1970), 276).
Ein auch kommerziell erhältliches Trägersystem ist das vernetzte Copolymerisat aus Acrylamid bzw. Methacrylamid und Glycidylacrylat bzw. -methacrylat und/oder Allylglycidylether, das bevorzugt aus perlförmigen Partikeln besteht. Solche Matrixpolymerisate werden in der DE-C 27 22 751 bzw. der US-A 4 190 713 sowie der US-A 4 511 694 angegeben. Die Epoxyfunktionen eines solchen Trägersystems können unmittelbar mit nucleophilen Gruppen an der Proteinoberfläche kondensiert werden, sie können aber auch mittels Dicarbonsäurebishydraziden als bifunktionelle Reagentien verknüpft werden. (GB-A 2 212 500).
Von erheblichem praktischem Interesse sind ferner Membran-Matrix-Materialien als Träger von biologisch wirksamen Proteinen, insbesondere von immunologisch relevanten Proteinen oder von Enzymen.

Aufgabe und Lösung

Die vorliegende Erfindung hat sich die Verbesserung der Gebrauchseigenschaften von festen Trägermaterialien, insbesondere der vorstehend genannten Typen zur Aufgabe gesetzt. Es sollten also nicht so sehr grundsätzlich neue Trägertypen zu Verfügung gestellt werden als durch Modifikation bestehender Strukturen, deren Bindungsfähigkeit und Bindungseigenschaften verbessert werden.
Die bestehende Aufgabe sei am Beispiel der aus (Meth)acrylamid und Glycidyl(meth)acrylaten aufgebauten Trägerpolymeren sowie der Membran-Matrix-Polymeren näher erläutert.
Die oben beschriebenen vernetzten Copolymerisate aus (Meth)Acrylamid und Glycidyl(meth)acrylat bzw. Allylglycidylether haben sich als wertvolle Trägersysteme für verschiedene Isolierungs- und Reinigungsoperationen in der Biochemie erwiesen. (Vgl. E.L.V. Harris and S. Angal in Protein Purification Methods, pp. 261 - 269, I.R.L. Press 1989).
Die ausgezeichneten Gebrauchseigenschaften dieses Trägers ließen es wünschenswert erscheinen, auf diesem Trägergerüst aufbauend dessen Effizienz noch zu verbessern. Es stellte sich z.B. die Aufgabe, die Bindungsraten biologisch wirksamer Proteine an das Trägersystem zu erhöhen, möglichst ohne dafür andere Nachteile wie verminderte Stabilität bzw. größere Empfindlichkeit unter Reaktionsbedingungen in Kauf nehmen zu müssen.
Es wurde nun gefunden, daß die gestellte Aufgabe durch die erfindungsgemäß modifizierten festen Träger in hervorragender Weise gelöst werden kann.
Die Erfindung betrifft modifizierte, feste Träger T, die mindestens eine zur Fixierung von biologisch wirksamen Stubstanzen, insbesondere von Proteinen ausreichende Zahl von funktionellen Gruppen der Formel I

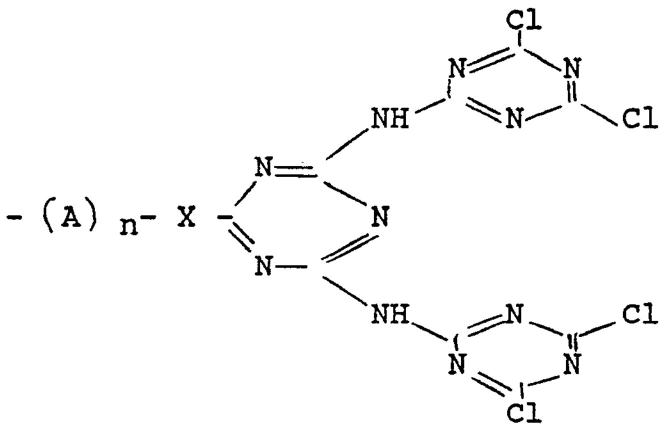

(I)

worin A für eine Spacergruppe, X für 0, S oder -NH und n für null oder eins steht,

in kovalenter Bindung an das Trägermaterial gebunden, enthalten. Sofern A als Spacergruppe in I enthalten ist, stellt sie eine mindestens zweigliedrige und bis sechsgliedrige Alkylen- oder eine Arylengruppe dar, die Bestandteil des festen Trägers selbst sein kann oder nachträglich mit dem festen Träger verknüpft worden sein kann. Diese Verknüpfung kann z.B. durch Alkylierung, Veresterung, Amidbildung oder Veretherung erfolgen (Vgl. H. J. Rehm and G. Reed, Vol., 7a, Enzyme Technology, Chapter 7, 347 - 384 ff, VCH 1987).

Besonders bevorzugt ist A mit der Bedeutung

$$-(CH_2-\underset{\displaystyle OH}{\underset{|}{CH}}-CH_2)-\text{und } X = NH.$$

Die modifizierten festen Träger werden allgemein ausgehend von an sich bekannten Trägermaterialien T', die eine ausreichende Zahl von nucleophil reagierenden Gruppen-XM enthalten, wobei X die für Formel I angegebene Bedeutung besitzt und M für Wasserstoff oder im Falle X = O oder S auch für ein Metallkation, insbesondere ein Alkalikation, bevorzugt Natrium oder Kalium steht, in einer mindestens dreistufigen Reaktion bestehend aus der Umsetzung mit einer ausreichenden Menge von 2,4,6-Trichlor-s-triazin, gefolgt von Substitution der Chlorgruppen am Triazin durch Aminogruppen und erneuter Umsetzung der so erhaltenen zweifach aminsubstituierten Triazingruppen, mit 2,4,6-Trichlor-s-triazin hergestellt. Die Herstellung der erfindungsgemäßen, modifizierten festen Träger T sei wiederum anhand eines Trägermaterials T'mit einem Gehalt an Glycidylgruppen dargestellt. Diese besonders vorteilhafte Ausführungsart betrifft ein Verfahren zur Herstellung von festen Trägern T mit einem Gehalt an funktionalen Gruppen, ausgehend von einem Trägermaterial T', welches eine Anzahl Glycidylgruppen der Formel II

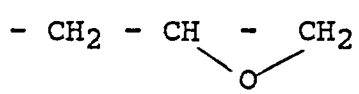

(II)

als funktionale Gruppen besitzt,

wobei in erster Stufe das Trägermaterial T' mit Ammoniak umgesetzt wird unter Umwandlung der Glycidylgruppen der Formel II in $\alpha$-Hydroxy-$\beta$-aminogruppen der Formel III

$$- CH_2 - \overset{\displaystyle OH}{\overset{|}{CH}} - CH_2NH_2 \qquad \text{(III)}$$

(Verbindung T'-III) und in zweiter Stufe die $\alpha$-Hydroxy-$\beta$-aminogruppen der Formel III unter HCl-Abspaltung mit 2,4,6-Trichlor-s-triazin zu N-Triazinyl-substituierten Gruppen der Formel IV

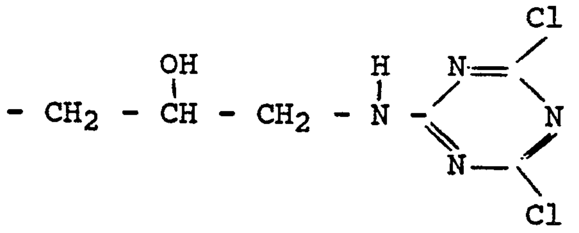

(IV)

(Verbindung T'-IV) umgesetzt werden, die in einer dritten Stufe durch Behandlung mit Ammoniak in aminsubstituierte Gruppen der Formel V umgewandelt werden

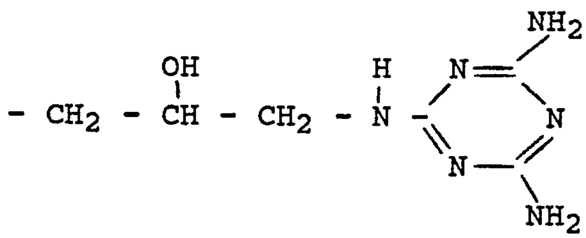

(V)

(Verbindung T'-V) welche durch Umsetzen mit mindestens zwei Äquivalenten 2,4,6-Trichlor-s-triazin zu den Gruppen der Formel (I) reagieren.

Besonders bevorzugt sind modifizierte feste Träger T-(I), die nach dem vorstehenden Verfahrensschema erhalten werden und bei deren Herstellung von den oben beschriebenen vernetzten Copolymerisaten aus Methacrylamid /Acrylamid und Glycidylacrylat/-methacrylat, vorzugsweise in Perlform als Matrixpolymerisat ausgegangen worden war. Als vernetzendes Monomer wird dabei vorzugsweise N,N-Methylenbis-methacrylamid eingesetzt.

Die Struktur der Matrixpolymerisate kann schematisch durch die folgende Formel T'-(II) wiedergegeben werden (vgl. E.L.V. Harris and S. Angal, loc.cit. S. 261 - 262).

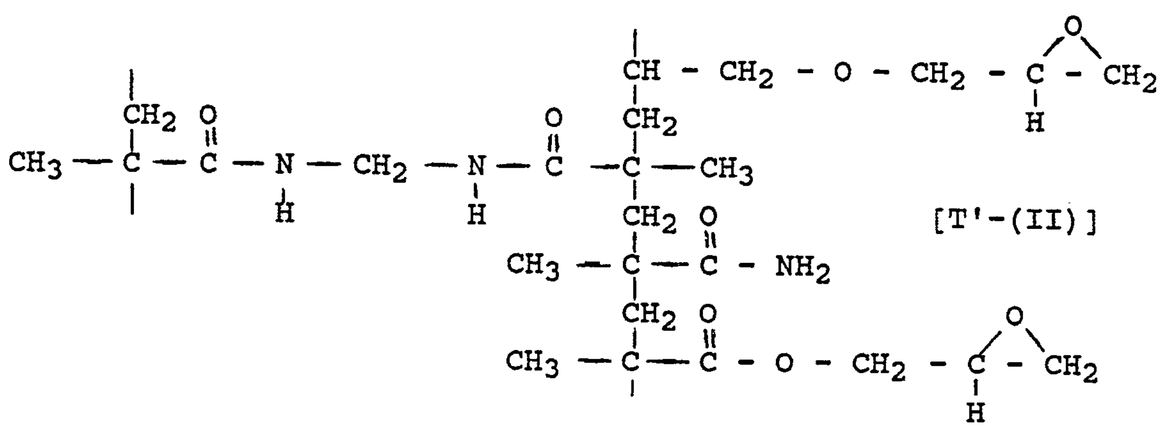

[T'-(II)]

Derartige Matrixpolymerisate werden in der DE-C 27 22 751 bzw. US-A 4 190 713 sowie der US-A 4 511 694 beschrieben. Die Perlen besitzen in der Regel einen Durchmesser von 5 - 1 000 $\mu$m, insbesondere 30 - 1 000 $\mu$m und weisen einen inneren Hohlraum (Hohlperlen) auf. Als Anhalt für den Gehalt an Glycidylgruppen im Matrixpolymerisat, die mit Ammoniak zur Raktion gebracht werden können, sei ein Wert von 0,8 - 2,5 $\mu$ Mol pro mg trockenes Trägermaterial genannt. Weitere Kenndaten für ein handelsübliches Matrixpolymerisat (EUPERGIT C der Röhm GmbH), das für Matrixpolymerisate des angegebenen Typs repräsentativ ist, lassen sich der folgenden Tabelle entnehmen.

| Kenndaten | Spezifikation |
|---|---|
| - Mittlere Korngröße | 140 - 180 um |
| - Porendurchmesser: | 40 nm |
| - Ausschlußgrenze $=M_{LIM}$: | $2.10^5$ Daltons |
| - Bindungsaktive Oberfläche: | 180 $m^2/g$ (trocken) |
| - Epoxidgehalt: | 800 - 1000 µmol/g (trocken) |
| - Wasseraufnahme: | 2,3 ml/g (trocken) |
| - Dichte = $d_4^{20}$: | 1,20 |
| - Schüttdichte: | 0,34 g/ml |
| - Bindungskapazität: (bei üblichen Bedingungen) | |
| Humanalbumin: | 48 mg/g (feucht) |
| Human IgG: | 34 mg/g (feucht) |
| - Quellverhalten gegenüber Wasser: | 1 : 1,4<br>1 ml (trocken) ergibt 1,4 ml (feucht) |
| - Löslichkeit (in Wasser, Puffern und organischen Lösungsmitteln): | unlöslich |
| - Druckstabilität | 300 bar |

Unter dem Elektronenmikroskop erkennt man die makroporöse Struktur der Perlen mit Kanälen und Hohlräumen, die einen Durchmesser von 0,1 - 2,5 µm (1000 - 25000 Å) besitzen, d.h. in einer solchen Größenordnung, daß Enzym- bzw. Substratmoleküle mit einer Größe von 10 - 100 Å das gesamte Innere der makroporösen Matrix erreichen können.

Anstelle der oben spezifizierten Matrixpolymerisate können auch solche angewendet werden, die bei grundsätzlich gleichartigem chemischen Aufbau wie in Formel T'-(II) eine durchschnittlicher Porengröße von 30 - 80 µm besitzen. Solche Produkte sind unter der Bezeichnung EUPERGIT C 30 N im Handel. Desweiteren sind Matrixpolymerisate mit grundsätzlich gleichartigem chemischen Aufbau aber einer Porengröße im Bereich 200 - 250 µm geeignet, die unter dem Namen EUPERGIT C 250 L im Handel sind. Weitere brauchbare Matrixpolymerisate weisen grundsätzlich gleiche chemische Zusammensetzung wie in Formel T'-(II) auf, sind aber kompakt, d.h. besitzen keinen wesentlichen Porenanteil. Produkte mit einer mittleren Korngröße von ca. 0,6 - 1,4 µm sind unter der Bezeichnung EUPERGIT CIZ im Handel erhältlich. Weitere Angaben zu den genannten Handelsprodukten finden sich in der Literatur. Weiter können auch oberflächenreiche Systeme (CA-A 1 212 058) und Kernschale-Latices (US-A 4 829 101) als Ausgangsmaterialien dienen.

Wie bereits erwähnt sind auch andere Typen von Matrixmaterialien als Ausgangsmaterial zur Herstellung der modifizierten festen Träger T geeignet, insbesondere solche, bei denen die Anwesenheit von Glycidylgruppen der Formel II im Ausgangsmaterial die Voraussetzung bietet. Dazu gehören z.B. Polysaccharide wie Cellulose, Agarose, Sepharose.

Die Glycidyl-(=Epoxy)-gruppen II können z.B. auch über eine Reaktion mit 1,4-bis(2,3-Epoxypropoxy)butan in die Polysaccharide eingebracht werden (vgl. Dechema Monographs No. 1724 - 1731, Vol. 84, "Characterization of Immobilized Biocatalysts", Ed. K. Buchholz, Verlag Chemie 1979; P. Vretblad, FEBS Left, 47 (1974) 86).

Weiter sind - wie bereits erwähnt - Trägermaterialien T von besonderem Interesse, die Aminogruppen

tragende Membrane darstellen. Derartige Membrane können beispielsweise Aminogruppen und Carboxylgruppen gleichzeitig enthalten, d.h. amphoter ausgebildet sein. Beispielsweise kann ein derartiges Membran-Matrixmaterial ein Polykondensationsprodukt aus Hexandiamin und Adipinsäure nach Art des Nylon PA66 darstellen. Derartige Polymerisate lassen sich durch die Formel VI

$$HOOC-(CH_2)_4-CO-[NH-(CH_2)_6-NH-CO-(CH_2)_4-CO]_m-NH-(CH_2)_6-NH_2 \quad (VI)$$

darstellen, wobei - als Anhalt - m im Bereich 80 - 100 liegt.
Geeignete Membranmaterialien auf dieser Basis sind z.B. unter dem Namen BIODYNE A (Amphoteric Nylon 66) der Fa. PALL, Bio Support Division Portsmouth, UK erhältlich.

Die biologisch signifikanten Proteine

Die biologisch signifikanten Proteine, zu deren kovalenter Fixierung die erfindungsgemäßen festen Träger T dienen, sind in allgemeiner Form bereits vorstehend charakterisiert worden (vgl. Stand der Technik).

Sie weisen in der Regel nucleophile Gruppen, neben OH- und SH-Gruppen speziell Aminogruppen auf, die mit den funktionellen Gruppen (s. Formel I) der Träger T unter biologisch akzeptablen Bedingungen reagieren. Genannt seien Biokatalysatoren wie Enzyme, sowie Zellen, Organellen und immunologisch aktive Materialien und Strukturen, insbesondere Antikörper. Die immobilisierten Biokatalysatoren können z.B. zur Produktion bzw. der Umwandlung sehr verschiedenartiger Substrate wie Aminosäuren, Peptide und Enzyme, von Zuckern, organischen Säuren, Antibiotika, Steroiden, Nucleosiden und Nucleotiden, Lipiden, Terpenoiden und organischen Grundchemikalien dienen (vgl. Ullmann 5. Auflage, Bd. 9A, loc.cit. S. 389 - 390).
Als immunologisch aktive Strukturen seien z.B. Mikroorganismen, wie gram-positive und gram-negative Bakterien, Spirochäten, Mycoplasma, Mycobacterien, Vibrionen, Actinomyceten, Protozoen, wie intestinale Protozoen, Amöben, Flagellaten, Sporozoen, intestinale Nematoden und Gewebenematoden (Würmer), Trematoden (Schistosomen, Egel), Cestoden, Toxoplasma, sowie Pilze, wie Sporotrichum, Cyptocoecus, Blastomyces, Histoplasma, Coccidioides, Candida, Viren und Rickettsien, wie Hunde-Hepatitis, Shope-Papillome, Influenza A + B, Hühnerpest, Herpes-Simplex, Adenoviren, Polyane, Rous-Sarkom, Impfpocken, Poliovirus, Masern, Hundestaupe, Leukämie, Mumps, Newcastle-Krankheit, Sendai, ECHO, Maul- und Klauenseuche, Psittacosis, Rabies, Extromelia, Baumviren, weiter Gewebe-Antigene, Enzyme, wie Pancreas-Chymotrypsinogen, Procarboxypeptidase, Glucose-Oxidase, Lactatdehydrogenase, Uricase, Aminosäure-Oxidase, Urease, Asparaginase, Proteasen, Blutzellen-Antigene, Blutgruppensubstanzen und andere Isoantigene, wie Blutplättchen, Leucozyten, Plasma-Proteine, Antikörper, einschließlich Auto-Antikörper genannt. Erwähnt seien insbesondere monoklonale Antikörper, die gegen Antigene bakteriellen oder viralen Ursprungs, gegen Autoimmun-Antigene, sowie gegen Tumor-Antigene, Gewebe-Antigene und sonstige gerichtet sind.
Von besonderem Interesse ist beispielsweise die kovalente Fixierung von "Protein A".

Unter "Protein A" wird wie üblich ein Zellwandprotein, das zur Bindung von Immunglobulin G zuständig ist, beispielsweise aus Staphylococcus aureus verstanden (vgl. Protein A from Staphylococcus aureus. Its isolation by affinity chromatography and its use as an immunosorbent for isolation of immunoglobulins. Hjelm H. et al. FEBS Lett. 28, 73 - 76 (1972)).
Weiter seien als zu immobilisierende Proteine besonders erwähnt: Protein G, Protein A/G, Trypsin-Inhibitor, Anti-IgG, monoklonale und polyklonale Antikörper, sowie die Enzyme Pepsin und Papain.

Herstellung der modifizierten Träger T

Die Herstellung der modifizierten, festen Träger T sei am Beispiel des Trägers T'-(I) näher erläutert:
Das feste Trägermaterial T'welches eine Anzahl Glycidylgruppen der Formel II als funktionelle Gruppen besitzt entsprechend den vorstehenden Angaben, insbesondere solche vom Typ T'-(II) (EUPERGIT C) wird in einem geeigneten Gefäß vorgelegt.

Dazu gibt man bei Raumtemperatur Ammoniak, z.B. einmolaren wäßrigen Ammoniak in beispielsweise solchen Mengen, daß auf 1 g trockenes Matrixmaterial T'-(II) 4 ml wäßr. Ammoniak kommen. Man läßt vorzugsweise über mehr als einen Tag, als Anhaltspunkt über ca. 72 Stunden einwirken, zweckmäßigerweise unter leichtem Schütteln und wäscht das Gel mit destilliertem Wasser. Als zweckmäßige Maßnahme hat sich die Behandlung mit Mercaptoethanol zur Absättigung restlicher Epoxygruppen erwiesen. Man inkubiert über einige Stunden, zweckmäßig über Nacht (ca. 12 Stunden) unter leichtem Schütteln bei Raumtemperatur. Daraufhin wird das Gel mit destilliertem Wasser gewaschen und auf der Fritte unter Waschen mit z.B. Aceton getrocknet. Das Gel läßt sich im Vakuumtrockenschrank vorzugsweise bei 35 bis 45 Grad C vollständig trocknen [Funktionale Gruppen entsprechen der Formel III = Verbindung der Formel T'-(III)]. Zu dem so erhaltenen, getrockneten Matrix-Polymerisat in einem geeigneten Gefäß gibt man einen inerten alkalischen Puffer, z.B. Natriumcarbonatpuffer vom pH 10,8 ("NC-Puffer") in Mengen von etwa 10 ml Puffer auf 1 g des Matrix-Polymerisats und gibt dazu tropfenweise unter gelindem Rühren und bei Raumtemperatur das 2,4,6-Trichlor-s-triazin ("Trichlortriazin") in einem geeigneten trockenen inerten Lösungsmittel wie Aceton vorzugsweise so, daß etwa 0,15 g Trichlortriazin in ca. 2 ml Lösungsmittel auf 1 g des aminsubstituierten Matrixpolymerisats [= Formel T'-(III)] entfallen. Man inkubiert einige Minuten, beispielsweise ca. 10 Minuten bei Raumtemperatur zweckmäßig unter leichtem Schütteln und stellt den pH-Wert mit 0,1 M NC-Puffer auf pH 10,8 nach. Das Produkt wird dann auf einer Glassäule (mit Fritte) mittels eines inerten Lösungsmittels, beispielsweise mit 50 %-igem wäßrigen Aceton gewaschen und zweckmäßig trockengesaugt. [= Produkt T'-(IV)]
Zu diesem Produkt in einem geeigneten Gefäß gibt man 25 %-igen wäßrigen Ammoniak, vorzugsweise in solchen Mengen, daß 10 ml wäßriger Ammoniak auf 1 g des getrockneten aminsubstituierten Matrixpolymerisats kommen, inkubiert über einen gewissen Zeitraum, beispielsweise 30 min bei erhöhter Temperatur, beispielsweise im Bereich von 55 bis 65 Grad C, vorzugsweise bei ca. 60 Grad C unter leichtem Schütteln. Daraufhin wäscht man mit Wasser, vorteilhaft portionsweise mit zweckmäßig insgesamt 100 ml Wasser und anschließend mit Aceton (ca. 5 ml) und saugt das gebildete Gel (dessen funktionelle Gruppen der Formel V entsprechen) trocken. Zu dem getrockneten Gel gibt man 0,1 M NC-Puffer, beispielweise 10 ml und anschließend tropft man langsam (ca. 2 Minuten) Trichlortriazin in acetonischer Lösung zu, so daß auf 1 g des ursprünglichen aminsubstituierten Matrix-Polymerisats [= Formel T'-(III)] 0,3 g Trichlortriazin, vorzugsweise in ca. 2 ml Aceton kommen. Man inkubiert kurze Zeit, vorzugsweise ca. 8 min bei Raumtemperatur unter leichtem Schütteln und stellt anschließend den pH-Wert mittel NC-Puffer auf pH 10,8 nach.
Das gebildete Gel wird zweckmäßig mit 50 %-igem (Vol.) wäßrigem Aceton, vorzugsweise ca. 100 ml, gewaschen, trocken gesaugt und gefriergetrocknet. Das so erhaltene Matrixpolymerisat, das mit funktionalen Gruppen der Formel I besetzt ist [= Matrixpolymerisat T'-(I)] kann als solches bei trockener Lagerung, gegebenenfalls auch unter Kühlung aufbewahrt oder unmittelbar verwendet werden.

Bei der Verwendung von Gruppen-XM (siehe vorstehende Definitionen) insbesondere Aminogruppen tragenden Membranmaterialien als Trägermaterial T' kann beispielsweise wie folgt vorgegangen werden. Man legt das Membranmaterial vor und fügt das 2,4,6-Trichlor-s-triazin vorzugsweise gelöst in einem geeigneten, inerten Lösungsmittel wie beispielsweise Nitromethan, bevorzugt tropfenweise zu. Dann wird zweckmäßig relativ kurzzeitig, etwa 10 min bei Raumtemperatur und unter leichtem Schütteln inkubiert. Dann wäscht man mit Lösungsmittel wie beispielsweise Nitromethan nach und trocknet das Membranmaterial beispielsw eise an der Luft.
Zu dem getrockneten Reaktionsprodukt gibt man Ammoniak, beispielsweise in Form 25 %-igem wäßrigen Ammoniaks und inkubiert damit, vorzugsweise bei erhöhter Temperatur beispielsweise bei 60 Grad C und im Bereich von 1/2 Stunde, zweckmäßig unter leichtem Schütteln. Anschließend wird mit Wasser gewaschen und erneut getrocknet. Anschließend wird erneut eine ausreichende Menge 2,4,6-Trichlor-s-triazin zugegeben, vorzugsweise in Lösungsmittel gelöst und zweckmäßig durch langsames Zutropfen. Man inkubiert wiederum kurzzeitig, etwas über 8 Minuten und bei Raumtemperatur und unter leichtem Schütteln. Dann wäscht man mit Lösungsmittel nach. Zweckmäßig wird das modifizierte Trägermaterial anschließend luft- und dann gefriergetrocknet.

Immobilisierung der Proteine

Das modifizierte feste Trägermaterial T, bevorzugt das Matrixpolymerisat T'-(I), wird vorgegeben. Aus dem (in der Regel bekannten) Gehalt der Ausgangsmaterialien vom Typ T'-(II) an den funktionellen Gruppen der Formel II kann in erster Näherung auf den Gehalt an bindungsfähigen Einheiten der Formel I im Endprodukt T'-(I) geschlossen werden. Als Anhalt für die anzusetzende Quantität sei ein Verhältnis: 0,25 g T'-(I) auf 5 und bis 10 mg des zu immobilisierenden Proteins angegeben. Man gibt zweckmäßig zu dem Trägermaterial einen geeigneten Puffer, vorzugsweise als Standard-Phosphat-Puffer, "Phosphate Buffered Saline" (PBS) in Mengen von etwa der Hälfte des Volumens des Träger-Gels. (Zum PBS. Vgl. Sigma-Katalog). Anschließend gibt man das zu immobilisierende Protein in Lösung bzw. Suspension zu und inkubiert über einige Stunden, beispielsweise über Nacht, vorzugsweise unter leichtem Schütteln. Danach

wird vorzugsweise mit geeignetem Puffer, z.B. PBS gewaschen, anschließend mit 10 Vol.-% Ethanolamin, aufgenommen in PBS-Pufferlösung mit einem pH-Wert von 8,0 abgesättigt und unter leichtem Schütteln einige Zeit, beispielsweise 4 Stunden, bei Raumtemperatur gehalten. Abschließend wird das Gel zur vollständigen Entfernung des Ethanolamins mit PBS gewaschen.
Das immobilisierte Protein kann bei 4 Grad C in PBS zweckmäßig mit einem geeigneten Konservierungsmittel, beispielsweise Thimerosal in z.B. 0,02 Gew.-% aufbewahrt werden.
Grundsätzlich ähnlich gestaltet sich auch die Beladung des vorstehend beschriebenen, modifizierten Membranmaterials mit Protein.
Dabei wird das Membranmaterial mit Standard-Phosphat-Puffer (PBS) versetzt und das Protein zugegeben. Zweckmäßig inkubiert man mehrere Stunden, beispielsweise über Nacht bei Raumtemperatur und unter leichtem Schütteln. Dann wird mit PBS gewaschen und mit einer 10 %-igen Ethanolaminlösung (pH 8,0) abgesättigt. Nach einer gewissen Zeit, beispielsweise 4 Stunden unter leichtem Schütteln wird das nunmehr beladene Membranmaterial mit PBS gewaschen und steht zur weiteren Verwendung zur Verfügung.

Vorteilhafte Wirkungen

Die vorteilhaften Wirkungen der modifizierten, festen Träger T, die funktionale Gruppen der Formel I enthalten, insbesondere T'-(I)-1 und T'-(I)-2, leiten sich u.a. aus der Tatsache ab, daß statt der einen, zur kovalenten Bindung befähigten Funktion eines gegebenen Trägermaterials, beispielsweise der Epoxy-Gruppe der Trägermaterialien T', nunmehr vier reaktive Chloratome zur Verfügung stehen (was nicht bedeutet, daß stets alle Chloratome an der Bindung beteiligt sind). Die Bindungskapazität für Proteine wird dadurch drastisch gesteigert, wie aus dem Vergleich (vgl. Tabelle 1) deutlich wird. Als Ausgangsmaterial stehen eine Vielzahl von Trägermaterialien T', die Gruppen-XM enthalten oder in solche umgewandelt werden können, zur Verfügung.

Die folgenden Beispiele dienen zur Erläuterung der Erfindung.

BEISPIELE

1. Ausgehend von einem Epoxygruppen-haltigen Trägermaterial T'.

A-1 Umsetzung des Trägermaterials T'-(II) zu T'-(III) mit Ammoniak

1 g Trägermaterial T'-(II) (® Eupergit C) vorlegen Zugabe von 4 ml 1 M $NH_4OH$
Inkubation 72 Stunden bei Raumtemperatur, leicht schütteln Gel mit destilliertem Wasser waschen
Gel absättigen mit 5 % Mercaptoethanol pH 8,0
Inkubation über Nacht bei Raumtemperatur, leicht schütteln Gelansatz mit destilliertem Wasser waschen
Gel mit Aceton über Fritte trocknen
Gel über Nacht im Vakuumschrank bei ca. 40 Grad C vollständig trocknen

B. Umsetzung des Trägermaterials T'-(III) zu T'-(I)

1 g Trägermaterial T'-(III) aus A-1
Zugabe von 10 ml 0,1 M Natriumcarbonatpuffer (NC) pH 10,8 0,15 g Trichlortriazin in 2 ml Aceton lösen und tropfenweise zu Eupergit-$NH_2$ geben
Inkubation 10 min bei Raumtemperatur, leicht schütteln nachstellen des pH-Wertes mit NC-Puffer auf pH 10,8 waschen in Bio Rad-Säule (Glas) mit 50 % Aceton trocken saugen, (= Trägermaterial T'-(IV)-1)
Zugabe von 10 ml 25 % Ammoniak
Inkubation 30 min bei 60 Grad C, leicht schütteln, waschen mit Wasser
waschen mit Aceton,
Gel trocken saugen,
Zugabe von 10 ml 0,1 M Natriumcarbonatpuffer pH 10,8, 0,3 g Trichlortriazin in Aceton lösen und langsam zutropfen,
Inkubation 8 min bei Raumtemperatur, leicht schütteln, pH-Wert mit NC-Puffer pH 10,8 nachstellen, Gel waschen mit 50 %-igem wäßrigem Aceton trocken saugen,
gefriertrocknen, (= Trägermaterial T'-(I)-1)

C-1 Kopplung von Protein A an Trägermaterial T'-(I)

0,25 g Trägermaterial T'-(I) aus B. (= 1 ml Gel),
Zugabe von 500 µl PBS,
Zugabe von 1 ml Protein A (5 mg/ml),
Inkubation über Nacht bei Raumtemperatur, leicht schütteln waschen mit PBS,
absättigen mit 10 % Ethanolamin pH 8,0/PBS,
4 Stunden bei Raumtemperatur, leicht schütteln,
Gel in PBS waschen,

2. Ausgehend von Membranmaterial als Trägermaterial T'.

B-2. Umsetzung des $NH_2$-haltigen Membranmaterials mit 2,4,6-Trichlor-s-triazin.

2 $cm^2$ Membranmaterial (BIODYNE A ®) vorlegen, tropfenweise Zugabe von 0,15 g Trichlortriazin in 2 ml Nitromethan gelöst. 10 min Inkubation bei Raumtemperatur unter leichtem Schütteln. Waschen mit Nitromethan, lufttrocknen. (= Trägermaterial T'-IV-2)
Zugabe von 10 ml 25 %-igem Ammoniak.
Inkubation 30 min lang bei 60 Grad C unter leichtem Schütteln. Waschen mit Wasser. Trocknen des Membranmaterials an der Luft, langsames Zutropfen von 0,15 g Trichlortriazin in 4 ml Nitromethan gelöst. 8 min Inkubation bei Raumtemperatur unter leichtem Schütteln. Membran mit Nitromethan waschen, lufttrocknen und abschließend gefriertrocknen (= Trägermaterial T'-(I)-2).

C-2. Kopplung von Protein A an das Trägermaterial aus B-2

2 $cm^2$ modifiziertes Membranmaterial aus B-2.
Zugabe von 500 µl PBS, gefolgt von der Zugabe von 1 ml Protein A (150 µg/ml).
Inkubation über Nacht bei Raumtemperatur unter leichtem Schütteln.
Waschen mit PBS.
Absättigen mit 10 % Ethanolamin pH 8,0/PBS, 4 Stunden bei Raumtemperatur bei leichtem Schütteln.
Membran in PBS waschen.
Die Vorteile, die durch die Kopplung des Proteins an die Träger T erreicht werden können, werden anhand der TABELLE 1 für Beispiel 1 und anhand der TABELLE 2 für Beispiel 2 aufgezeigt.

TABELLE 1

Bindungskapazität für Immunoglobulin G (IgG)

| Kopplungsmethode | Protein A immobilisiert pro g Gel (feucht) (mg) | IgG gebunden pro g Gel (feucht) (mg) |
|---|---|---|
| Stand der Technik ® EUPERGIT C unmodifiziert | 10 | 7 |
| Beispiel 1 Trägermaterial T'-(IV)-1 | 5 | 12 |
| Beispiel 1 Trägermaterial T'-(I)-1 | 5 | 20 |

TABELLE 2

Bindungskapazität für Immunoglobulin G (IgG)

| Kopplungsmethode | Protein A immobilisiert pro 2 $cm^2$ Membran | IgG gebunden pro 2 $cm^2$ Membran |
|---|---|---|
| Epoxygruppen-haltiges Membran-material/direkte Bindung | 150 µg | 100 µg |
| Beispiel 2 Trägermaterial T'-(IV)-2 | 150 µg | 375 µg |
| Beispiel 2 Trägermaterial T'-(I)-2 | 150 µg | 600 µg |

**Patentansprüche**

**1.** Modifizierte, feste Träger zur kovalenten Immobilisierung von Proteinen auf Basis von an sich bekannten Matrixmaterialien,

dadurch gekennzeichnet,

daß die modifizierten festen Träger T eine Mehrzahl von zur kovalenten Immobilisierung geeigneten

funktionellen Gruppen der Formel I

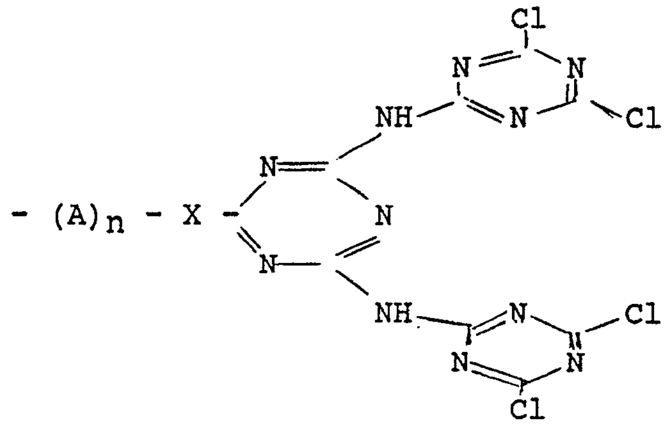

(I)

worin A für eine Spacergruppe, X für O, S, NH und n für null oder eins steht, besitzen, welche kovalent an das Matrixmaterial gebunden sind.

2. Modifizierte feste Träger gemäß Anspruch 1, dadurch gekennzeichnet, daß das Matrixmaterial ausgewählt ist aus der Gruppe bestehend aus vernetzten Copolymerisaten des Methacrylamids und des Acrylamids, oder modifizierten Polysacchariden vom Typ der Cellulose, Agarose, Sepharose.

3. Modifizierte feste Träger gemäß den Ansprüchen 1 und 2, dadurch gekennzeichnet, daß A in Formel I für eine

$$\text{Gruppe } -CH_2-\underset{\displaystyle OH}{\underset{|}{CH}}-CH_2- \text{ und X für } -NH \text{ steht.}$$

4. Modifizierte feste Träger gemäß den Ansprüchen 1 bis 3, dadurch gekennzeichnet, daß das Matrixmaterial ein Membranmaterial darstellt.

5. Verfahren zur Herstellung von modifizierten festen Trägern T mit einem Gehalt an funktionalen Gruppen der Formel I gemäß den Ansprüchen 1 bis 3, dadurch gekennzeichnet, daß man von einem Trägermaterial T', welches eine Anzahl kovalent gebundener Glycidylgruppen der Formel II

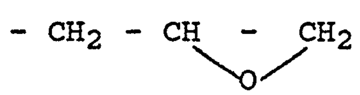

(II)

als funktionale Gruppen besitzen, ausgehend in erster Stufe das Trägermaterial T' mit Ammoniak umsetzt unter Umwandlung der Glycidylgruppen der Formel II in α-Hydroxy-β-aminogruppen der Formel III

$$-CH_2-\overset{\displaystyle OH}{\overset{|}{CH}}-CH_2NH_2 \qquad \text{(III)}$$

und in zweiter Stufe die α-Hydroxy-β-aminogruppen der Formel III unter HCl-Abspaltung mit 2,4,6-Trichlor-s-triazin zu N-Triazinyl-substituierten Gruppen der Formel IV

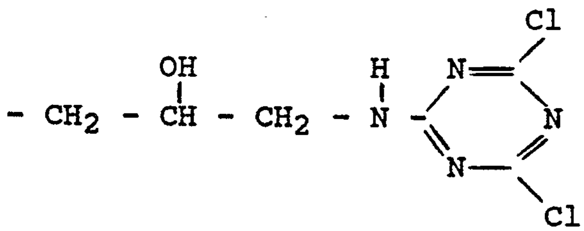

(IV)

umsetzt, die man in einer dritten Stufe durch Behandlung mit Ammoniak in aminsubstituierte Gruppen der Formel V umwandelt

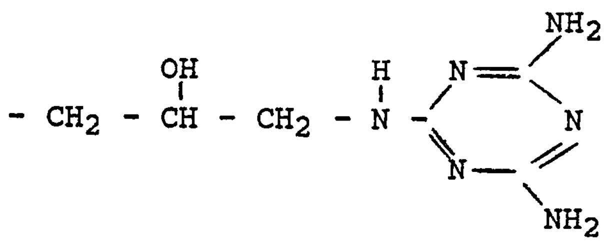

(V)

welche man in letzter Stufe durch Umsetzung mit mindestens zwei Äquivalenten 2,4,6-Trichlor-s-triazin zu den Gruppen der Formel (I) reagieren läßt.